# EUROPEAN PATENT SPECIFICATION

(11) **EP 2 769 270 B1**
(45) Date of publication and mention of the grant of the patent: **19.09.2018**
(21) Application number: 12798835.0
(22) Date of filing: 15.10.2012
(51) Int. Cl.: G03H 1/00, G03H 1/22, G06F 3/16, G06T 19/20

(54) **HOLOGRAPHIC USER INTERFACES FOR MEDICAL PROCEDURES**
HOLOGRAFISCHE BENUTZEROBERFLÄCHEN FÜR MEDIZINISCHE EINGRIFFE
INTERFACES UTILISATEUR HOLOGRAPHIQUES POUR PROCÉDURES MÉDICALES

(30) Priority: 20.10.2011 US 201161549273 P
(43) Date of publication of application: 27.08.2014
(73) Proprietor: Koninklijke Philips N.V., 5656 AE Eindhoven (NL)
(72) Inventor: VÉRARD, Laurent, NL-5656 AE Eindhoven (NL); CHAN, Raymond, NL-5656 AE Eindhoven (NL); RUIJTERS, Daniel Simon Anna, NL-5656 AE Eindhoven (NL); DENISSEN, Sander Hans, NL-5656 AE Eindhoven (NL); SLEGT, Sander, NL-5656 AE Eindhoven (NL)
(74) Representative: Steffen, Thomas
(86) International application number: PCT/IB2012/055595
(87) International publication number: WO 2013/057649

(56) References cited:
- WO-A1-2010/004563
- WO-A2-2005/010623
- US-A1- 2009 237 759

## Description

### CROSS-REFERENCE TO RELATED APPLICATIONS:

This application claims priority to U.S. provisional application number 61/549,273 filed on October 20, 2011, the entire disclosure of which is hereby incorporated herein by reference in its entirety.

### BACKGROUND:

### Technical Field

The present disclosure relates to medical systems, devices and methods, and more particularly to systems, devices and methods pertaining to integration of holographic image data with other information to improve accuracy and effectiveness in medical applications.

### Description of the Related Art

Auto-stereoscopic displays (ASDs) for three-dimensional (3D) visualization on a two-dimensional (2D) panel, without the need for user goggles/glasses, have been investigated. However, resolution and processing time limits the ability to render high quality images using this technology. Additionally, these displays have generally been confined to a 2D plane (e.g., preventing a physician from moving around or rotating the display to view the data from different perspectives). Although different perspectives may be permitted with a limited field of view, the field of view for this type of display still suffers from breakdown of movement parallax.

Similarly, user input for manipulation of data objects has largely been confined to mainstream 2D mechanisms, e.g., mice, tablets, keypads, touch panels, camera-based tracking, etc. Accordingly, there is a need for a system, device and method as disclosed and described herein which can be used to overcome the above-identified deficiencies.

WO 2005/010623 A2 discloses hologram production techniques which can combine source data representing realistic information describing an environment with source data providing representational information describing a feature of the environment and/or some object interacting with the environment. The combined data is used to produce holograms, and particularly holographic stereograms including features that enhance the visualization of the environment depicted in hologram. A haptic interface can be used in conjunction with such holograms to further aid use of the hologram, and to provide an interface to secondary information provided by a computer and related to the images depicted in the hologram.

US 2009/0237759 A1 discloses a display system for diagnostic image systems for reproducing three-dimensional medical images. The display system has at least one holographic projection facility that is connected to the diagnostic image system. The projection device produces a hologram of the three-dimensional medical images in an examination or intervention room.

WO2010/004563A1 discloses methods and systems for displaying images, and for implementing volumetric user interfaces, comprising a light source, an image producing unit which produces an image upon interaction with light approaching the image producing unit from the light source, an eyepiece and a mirror directing light from the image to a surface of the eyepiece, wherein the surface has a shape of a solid of revolution formed by revolving a planar curve at least 180° around an axis of revolution.

### SUMMARY

In accordance with the present principles, an interactive holographic display system includes a holographic generation module configured to display a holographically rendered anatomical image. A localization system is configured to define a monitored space on or
around the holographically rendered anatomical image. One or more monitored objects have their position and orientation monitored by the localization system such that coincidence of spatial points between the monitored space and the one or more monitored objects triggers a response in the holographically rendered anatomical image, wherein the response includes a haptic feedback to a user, the haptic feedback being incorporated by using ultrasound to generate vibrations in the air, wherein the localization system includes a shape sensing system for performing shape sensing of the one or more monitored objects, wherein the shape sensing system is a fiber optic based shape sensing system configured to include one or more optical fibers coupled to the one or more monitored objects, wherein a plurality of sensor elements are distributed over the length of the one or more optical fibers, wherein three or more fiber cores with the plurality of sensor elements are incorporated along the length of the one or more optical fibers embedded in the one or more monitored objects for determining a three-dimensional form of the one or more monitored objects.

Another interactive holographic display system includes a processor and memory coupled to the processor. A holographic generation module is included in the memory and configured to display a holographically rendered anatomical image as an in-air hologram or on a holographic display. A localization system is configured to define a monitored space on or around the holographically rendered anatomical image. One or more monitored objects has their position and orientation monitored by the localization system such that coincidence of spatial points between the monitored space and the one or more monitored objects triggers a response in the holographically rendered anatomical image, wherein the response includes a haptic feedback to a user, the haptic feedback being incorporated by using ultrasound to generate vibrations in the air, wherein the response in the holographically rendered anatomical image includes one or more of: translation or rotation of the holographically rendered anatomical image, magnification adjustment of the holographically rendered anatomical image, marking of the holographically rendered anatomical image and feedback generation, wherein the localization system includes a shape sensing system for performing shape sensing of the one or more monitored objects, wherein the shape sensing system is a fiber optic based shape sensing system configured to include one or more optical fibers coupled to the one or more monitored objects, wherein a plurality of sensor elements are distributed over the length of the one or more optical fibers, wherein three or more fiber cores with the plurality of sensor elements are incorporated along the length of the one or more optical fibers embedded in the one or more monitored objects for determining a three-dimensional form of the one or more monitored objects.

A method for interacting with a holographic display includes displaying a holographically rendered anatomical image; localizing a monitored space on or around the holographically rendered anatomical image to define a region for interaction; monitoring a position and orientation of one or more monitored objects by the localization system; determining coincidence of spatial points between the monitored space the one or more monitored objects; and if coincidence is determined, triggering a response in the holographically rendered anatomical image, wherein the response includes a haptic feedback to a user, the haptic feedback being incorporated by using ultrasound to generate vibrations in the air, wherein the localizing comprises using a shape sensing system to perform shape sensing of the one or more monitored objects, wherein the shape sensing system is a fiber optic based shape sensing system configured to include one or more optical fibers coupled to the one or more monitored objects, wherein a plurality of sensor elements are distributed over the length of the one or more optical fibers, wherein three or more fiber cores with the plurality of sensor elements are incorporated along the length of the one or more optical fibers embedded in the one or more monitored objects for determining a three-dimensional form of the one or more monitored objects.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a system for interfacing with holograms in accordance with exemplary embodiments;
FIG. 2 is a perspective view of a hologram rendered with a data map or overlay thereon in accordance with an illustrative embodiment;
FIG. 3 is a block diagram showing an illustrative process flow for displaying a data map or overlay in a holographic image in accordance with an illustrative embodiment;
FIG. 4 is a block diagram showing an illustrative system and process flow for displaying static or animated objects in a holographic image in accordance with an illustrative embodiment;
FIG. 5 is a diagram showing an illustrative image for displaying an objects menu for selecting a virtual objects during a procedure for display in a holographic image in accordance with an illustrative embodiment;
FIG. 6 is a block diagram showing an illustrative system for controlling a robot using a holographic image in accordance with an illustrative embodiment;
FIG. 7 is a block diagram showing an illustrative system which employs haptic feedback with a holographic image in accordance with an illustrative embodiment;
FIG. 8 is a diagram showing multiple views provided to different perspectives in an illustrative system for displaying a holographic image or the like in accordance with one embodiment;
FIG. 9 is a block diagram showing an illustrative system for controlling a robot remotely over a network using a holographic image in accordance with an illustrative embodiment; and
FIG. 10 is a flow diagram showing a method for interfacing with a hologram in accordance with an illustrative embodiment.

### DETAILED DESCRIPTION OF EMBODIMENTS

In accordance with the present principles, systems, devices and methods are described which leverage holographic display technology for medical procedures. This can be done using 3D holographic technologies (e.g., in-air holograms) and real-time 3D input sensing methods such as optical shape sensing to provide a greater degree of human-data interaction during a procedure. Employing holographic technology with other technologies potentially simplifies procedure workflow, instrument selection, and manipulation within the anatomy of interest. Such exemplary embodiments described herein can utilize 3D holographic displays for real-time visualization of volumetric datasets with exemplary localization methods for sensing movements in free space during a clinical procedure, thereby providing new methods of human-data interaction in the interventional suite.

In one exemplary embodiment, 3D holography may be used to fuse anatomical data with functional imaging and "sensing" information. A fourth dimension (e.g., time, color, texture, etc.) can be used to represent a dynamic 3D multimodality representation of the status of an object of interest (e.g., organ). A display can be in (near) real-time and use color-coded visual information and/or haptic feedback/tactile information, for example, to convey different effects of states of the holographically displayed object of interest. Such information can include morphological information about the target, functional information about the object of interest (e.g. flow, contractility, tissue biomechanical or chemical composition, voltage, temperature, pH, pO₂, pCO₂, etc.), or the measured changes in target properties due to interaction between the target and therapy being delivered. The exemplary 3D holographic display can be seen from (virtually) any angle/direction so that, e.g., multiple users can simultaneously interact with the same understanding and information. Alternatively, it is possible to simultaneously display different information to different users positioned in the room, such as by displaying different information on each face of a cube or polyhedron, for example.

In one embodiment, one could "touch" or otherwise interact with a specific region of interest in the 3D holographic display (e.g., using one or multiple fingers, virtual tools, or physical instruments being tracked within the same interaction space), and tissue characteristics would become available and displayed in the 3D hologram. Such "touch" can also be used to, e.g., rotate the virtual organ, zoom, tag points in 3D, draw a path and trajectory plan (e.g., for treatment, targeting, etc.), select critical zones to avoid, create alarms, and drop virtual objects (e.g., implants) in 3D in the displayed 3D anatomy.

Exemplary embodiments according to the present disclosure can also be used to facilitate a remote procedure (e.g., where the practitioner "acts" on the virtual organ and a robot simultaneously or subsequently performs the procedure on the actual organ), to practice a procedure before performing the actual procedure in a training or simulation setting, and/or to review/study/teach a procedure after it has been performed (e.g., through data recording, storage, and playback of the 3D holographic display and any associated multimodality signals relevant to the clinical procedure).

Exemplary embodiments according to the present disclosure are further described herein below with reference to the appended figures. While such exemplary embodiments are largely described separately from one another (e.g., for ease of presentation and understanding), one having ordinary skill in the art shall appreciate in view of the teachings herein that such exemplary embodiments can be used independently and/or in combination with each other. Indeed, the implementation and use of the exemplary embodiments described herein, including combinations and variations thereof, all of which are considered a part of the present disclosure, can depend on, e.g., particular laboratory or clinical use/application, integration with other related technologies, available resources, environmental conditions, etc. Accordingly, nothing in the present disclosure should be interpreted as limiting of the subject matter disclosed herein.

A real-time 3D holographic display in accordance with the present principles may include a real-time six degree of freedom (DOF) input via localization technology embedded into a data interaction device (e.g., a haptic device for sensory feedback). An imaging / monitoring system for multidimensional data acquisition may also be employed. Datalinks between the holography display, localization system / interaction device, and imaging / monitoring system may be provided for communication between these systems. In one embodiment, the display, feedback devices, localization devices, measurement devices may be employed with or integrated with a computational workstation for decision support and data libraries of case information that can be dynamically updated / recalled during a live case for training / teaching / procedure guidance purposes (e.g., for similar archived clinical cases relative to the procedure and patient undergoing treatment).

It should be understood that the present invention will be described in terms of medical instruments; however, the teachings of the present invention are much broader and are applicable to any systems that can benefit from holographic visualization. In some embodiments, the present principles are employed in tracking or analyzing complex biological or mechanical systems. In particular, the present principles are applicable to internal tracking procedures of biological systems, procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

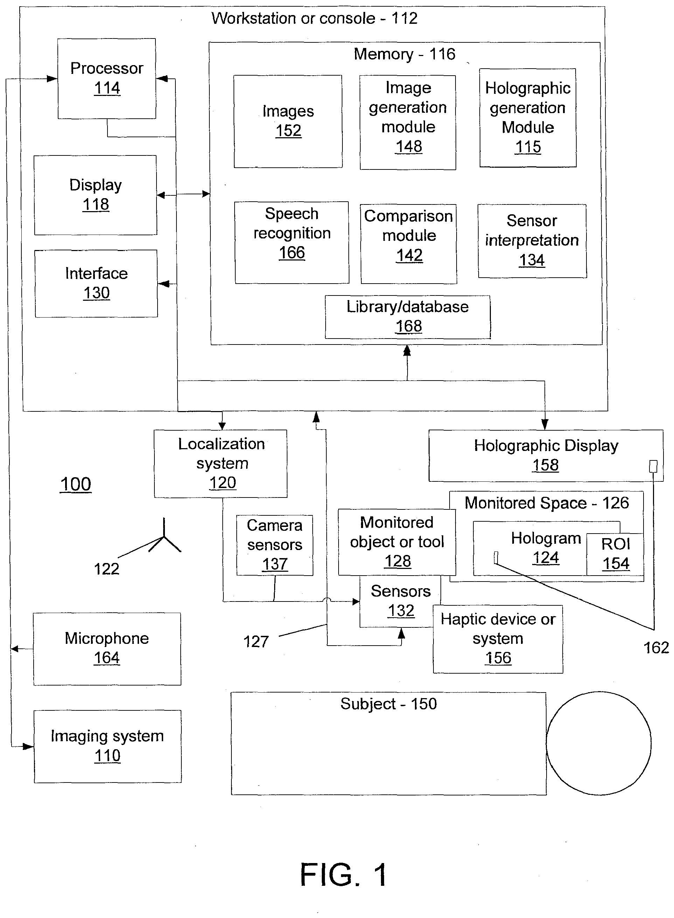

Referring now to the drawings in which like numerals represent the same or similar elements and initially to FIG. 1, a system 100 for generating and interacting with holographic images is illustratively shown in accordance with one embodiment. System 100 may include a workstation or console 112 from which a procedure is supervised and/or managed. Workstation 112 preferably includes one or more processors 114 and memory 116 for storing programs and applications. Memory 116 may store a holographic generation module 115 configured to render a holographic image on a display 158 or in-air depending on the application. The holographic generation module 115 codes image data to generate a three dimensional hologram. The coding may provide the hologram on a 2D display or in 3D media or 3D display. In one example, data from 3D imaging, e.g., computed tomography, ultrasound, magnetic resonance may be transformed into a hologram using spatial distribution and light intensity to render the hologram.

A localization system 120 includes a coordinate system 122 to which a holographic image or hologram 124 is registered. The localization system 120 may also be employed to register a monitored object 128, which may include virtual instruments, which are separately created and controlled, real instruments, a physician's hands, fingers or other anatomical parts, etc. The localization system 120 may include an electromagnetic tracking system, a shape sensing system, such as a fiber optic based shape sensing system, an optical sensing system, including light sensors and arrays, or other sensing modality, etc. The localization system 120 is employed to define spatial regions in and around the hologram or the holographic image 124 to enable a triggering of different functions or actions as a result of movement in the area of the hologram 124. For example, dynamic locations of a physician's hands may be tracked using a fiber optic shape sensing device. When the physician's hands enter the same space, e.g., a monitored space 126 about a projected hologram 124, the intensity of the hologram may be increased. In another example, the physician's hand movements may be employed to spatially alter the position or orientation of the hologram 124 or to otherwise interact with the hologram 124.

A monitored object or sensing system 128 may be spatially monitored relative to the hologram 124 or the space 126 around the hologram 124. The monitored object 128 may include the physician's hands, a real or a virtual tool, another hologram, etc. The monitored object 128 may include a sensor or sensors 132 adapted to monitor the position of the monitored object 128 such that when a position of the object or a portion thereof is within the hologram 124 or the space 126 around the hologram 124, a reaction occurs that is consistent with the type of the monitored object 128 and the action performed or to be performed by the monitored object 128. The sensor or sensors 132 may include EM sensors, fiber optic shape sensors, etc.

In one embodiment, the sensors 132 include fiber optic shape sensors. A sensor interpretation module 134 may be employed to interpret feedback signals from a shape sensing device or system (132). Interpretation module 134 is configured to use the signal feedback (and any other feedback, e.g., optical, electromagnetic (EM) tracking, etc.) to reconstruct motion, deflection and other changes associated with the monitored object 128, which may include a medical device or instrument, virtual tools, human anatomical features, etc. The medical device may include a catheter, a guidewire, a probe, an endoscope, a robot, an electrode, a filter device, a balloon device, or other medical component, etc.

The shape sensing system (132) may include one or more optical fibers which are coupled to the monitored object 128 in a set pattern or patterns. The optical fibers connect to the workstation 112 through cabling 127. The cabling 127 may include fiber optics, electrical connections, other instrumentation, etc., as needed.

Shape sensing system (132) may be based on fiber optic Bragg grating sensors. A fiber optic Bragg grating (FBG) is a short segment of optical fiber that reflects particular wavelengths of light and transmits all others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector.

A fundamental principle behind the operation of a fiber Bragg grating is Fresnel reflection at each of the interfaces where the refractive index is changing. For some wavelengths, the reflected light of the various periods is in phase so that constructive interference exists for reflection and, consequently, destructive interference for transmission. The Bragg wavelength is sensitive to strain as well as to temperature. This means that Bragg gratings can be used as sensing elements in fiber optical sensors. In an FBG sensor, the measurand (e.g., strain) causes a shift in the Bragg wavelength.

One advantage of this technique is that various sensor elements can be distributed over the length of a fiber. Incorporating three or more cores with various sensors (gauges) along the length of a fiber that is embedded in a structure permits a three dimensional form of such a structure to be precisely determined, typically with better than 1 mm accuracy. Along the length of the fiber, at various positions, a multitude of FBG sensors can be located (e.g., 3 or more fiber sensing cores). From the strain measurement of each FBG, the curvature of the structure can be inferred at that position. From the multitude of measured positions, the total three-dimensional form is determined.

As an alternative to fiber-optic Bragg gratings, the inherent backscatter in conventional optical fiber can be exploited. One such approach is to use Rayleigh scatter in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in three or more cores running within a single length of multicore fiber, the 3D shape and dynamics of the surface of interest can be followed.

In one embodiment, workstation 112 includes an image generation module 148 configured to receive feedback from the shape sensing system 132 or other sensor to sense interactions with the hologram 124. A position and status of the hologram 124 and its surrounding space 126 is known to the localization system 120. When the monitored object 128 enters the space 126 or coincides with the positions of the hologram 124, as determined by a comparison module 142, an action is triggered depending on a type of motion, a type of monitored object 128, a type of procedure or activity and/or any other criteria. The comparison module 142 informs the holographic generation module 115 that a change is needed. The holographic generation module 115 recodes the image data, which is processed and output to the image generation module 148, which updates the hologram 124 in accordance with set criteria.

In illustrative embodiments, the hologram 124 may include an internal organ rendered based on 3D images 152 of a patient or subject 150. The images 152 may be collected from the patient 150 preoperatively using an imaging system 110. Note the imaging system 110 and the patient 150 need not be present to employ the present principles as the system 100 may be employed for training, analysis or other purposes at any time. In this example, a physician employs a pair of gloves having sensors 132 disposed thereon. As the gloves/sensors 132, enter the space 126 and coincide with the hologram 124, the physician is able to rotate or translate the hologram 124. In another embodiment, the gloves include a haptic device 156 that provides tactile feedback depending on a position of the gloves/sensors relative to the hologram 124 or the space 126. In other embodiments, the haptic feedback is indicative of the tissue type corresponding with the hologram 124 and its representation. The haptic device or system 156 may include ultrasound sources, speakers or other vibratory sources to convey differences in state of the hologram 124 using vibrations or sound.

A display 118 and or display 158 may also permit a user to interact with the workstation 112, the hologram 124 and its components and functions, or any other element within the system 100. This is further facilitated by an interface 130 which may include a keyboard, mouse, a joystick, a haptic device, or any other peripheral or control to permit user feedback from and interaction with the workstation 112.

In one embodiment, a user (practitioner, surgeon, fellow, etc.) can touch (or otherwise interact with) a specific region of interest (ROI) 154 within the 3D holographic display 158 or the hologram 124 within the 3D holographic display (or elsewhere) to display additional information related to the selected specific region of interest, e.g., tissue characteristics, such as temperature, chemical content, genetic signature, pressure, calcification percent, etc. An overlay of information can be displayed or presented on a separate exemplary 2D display (118), whereby parts of the 2D display can be transparent, for example, for better viewing of displayed information. It is also possible that the exemplary 2D display 118 presents or displays other graphics and text in high resolution (e.g., in exemplary embodiments where the 3D display may be of relatively low or limited resolution).

Other embodiments can provide a practitioner (e.g., doctor) with a "heads up" display (as display 158) or as a combination display (118 and 158) to accommodate the display/presentation of such additional information. Additionally, other zones or regions of interest 154 can be automatically highlighted and/or outlined within the 3D holographic display 158 or hologram 124. Such other zones of interest can be, e.g., zones which have similar characteristics as the selected zone of interest and/or zones which are otherwise related.

According to yet another exemplary embodiment, the 3D holographic display 158 or hologram 124 may be employed with six degrees of freedom (6DOF) user tracking, e.g., with shape enabled instruments 132 and/or with camera based sensors 137, allowing for use as a user interface in 3D and real-time 6DOF user interaction. For example, a user (e.g., practitioner) is provided with the capability of touching a virtual organ being displayed as a 3D holographic image 124. The user can rotate, zoom in/out (e.g., changing the magnification of the view), tag points in 3D, draw a path and/or trajectory plan, select (critical) zones to avoid, create alarms, insert and manipulate the orientation of virtual implants in 3D in the anatomy, etc. These functions are carried out using the localization system(s) 120 and image generation system or module 148 working in conjunction with the holographic data being displayed for the hologram 124.

Seed points 162 maybe created and dropped into the 3D holographic display 158 or hologram 124 by touching (and/or tapping, holding, etc.) a portion of the display 158 or the hologram 124. The seed points 162 may be employed for, e.g., activation of virtual cameras which can provide individually customized viewing perspectives (e.g., orientation, zoom, resolution, etc.) which can be streamed (or otherwise transmitted) onto a separate high resolution 2D display 118.

The touch feature can by employed to create or drop virtual seed points 162 into the 3D display 158 for a plurality of tasks, e.g., initialization of segmentation, modeling, registration or other computation, visualization, planning step, etc. In addition to the 3D holographic display 158 or hologram 124 being used to display data of an anatomy, the display can also be used to display buttons, drop down menus, pointers/trackers, optional functions, etc. allowing users to interact and give commands to the system and/or any computer included therein or connected thereto (e.g., directly connected or via the Internet or other network).

In another embodiment, a microphone 164 may be employed to receive verbal information to connect, control, interact, etc. with the exemplary 3D holographic display 158 or hologram 124 via voice-controlled commands. A speech recognition engine 166 may be provided to convert speech commands into program commands to allow a user (e.g., surgeon) to interact with the 3D holographic display 158 or hologram 124 without having to use their hands. For example, a user could say "SHOW LAO FORTY", and the volume displayed within the holographic image would rotate to the proper angle to provide the user with the desired view. Other commands can range from those which are relatively simple, such as "ZOOM", followed by a specific amount e.g., "3 times" or so as to display particular (additional) information, to more complex commands, e.g., which can be related to a specific task or procedure.

According to another embodiment, a recording mode can be provided in memory 116 and made available to, e.g., play back a case on a same device for full 3D replay and/or on conventional (2D or 3D) viewing devices with automatic conversion of recorded 3D scenes into multiple 2D viewing perspectives (or rotating 3D models, e.g., in virtual reality modeling language (VRML)). Data connections between the holographic display 158 and recordings archived in a library/database 168 such as a picture archiving and communication system (PACS), Radiology Information Systems (RIS) or other electronic medical record system can be used to facilitate, e.g., visualization and diagnostic interpretation/data mining. Recordings can be replayed and used for, e.g., teaching and training purposes, such as to teach or train others in an individual setting, (e.g., when a user wants to review a recorded procedure performed), a small group environment (e.g., with peers and/or management), a relatively large class, lecture, etc. Such exemplary recordings may also be used for marketing presentations, research environments, etc. and may also be employed for quality and regulatory assessment, e.g., process evaluation or procedure assessment by hospital administrators, third-party insurers, investors, the Food and Drug Administration (FDA) and/or other regulatory bodies. Virtual cameras may be employed to capture or record multiple viewpoints/angles and generate multiple 2D outputs for, e.g., video capture or simultaneous display of images on different 2D television screens or monitors (or sections thereof).

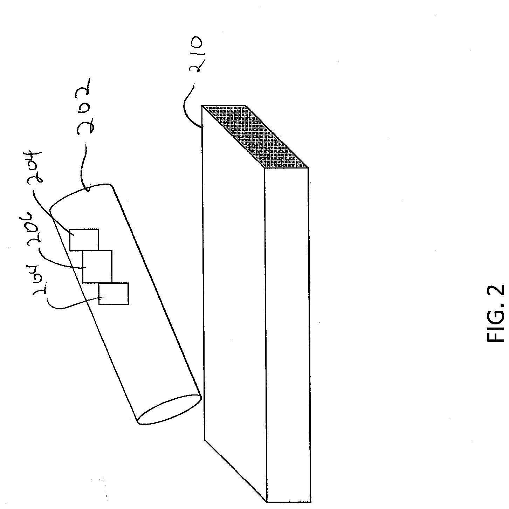

Referring to FIG. 2, in another embodiment, three-dimensional (3D) holography may be used to display volumetric data of an anatomy (e.g., from a 3D CT scan), for example, to fuse anatomical with functional imaging and "sensing" information, as well as temporal (time-related) information. The information may be employed to create (generate, produce, display, etc.) a dynamic 3D multimodality representation 202 (e.g., a hologram) of an object (e.g., organ) and a status thereof using visual indicators 204, 206, such as colors, contrast levels and patterns from a display 210. The object 202 (e.g., hologram 124) may show different regions 204, 206 to indicate useful data on the object 202. For example, epicardial and/or endocardial mapping data can be used to, e.g., display electrical activity data on a heart image during an electrophysiology procedure, superimposed with the anatomical imaging data of the heart (e.g., coming from CT, XperCT or MR). Another example is the display of temperature maps which can be provided by MR during ablation, or magnetic resonance high-intensity focused ultrasound (MR-HIFU) 4D (four-dimensional) information during an intervention (e.g., using MR digital data transfer systems and procedures). It is also possible to use information associated with a real-time radiation dose distribution map superimposed over the anatomical target during a radiation oncology treatment (Linac, brachytherapy, etc.), for example. Other embodiments are also contemplated.

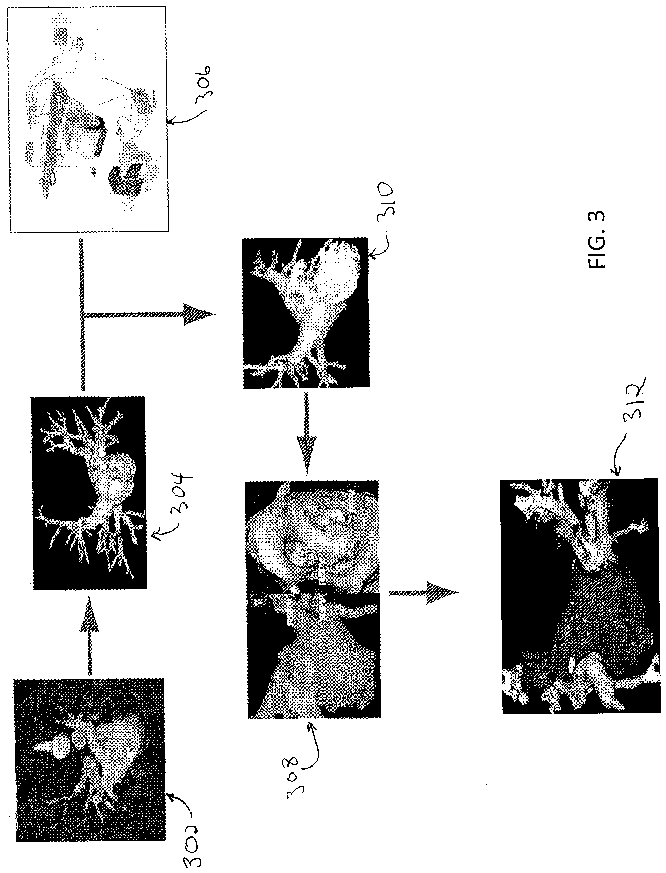

Referring to FIG. 3, an exemplary holographic visualization of functional and anatomical information, which may be employed during an interventional procedure in accordance with an exemplary embodiment, is illustratively shown. A volumetric image 302 of a heart, in this example, is acquired and may be segmented to reduce computational space and to determine anatomical features of the heart as opposed to other portions of the image. This results in a segmented image 304. Functional or device data is acquired by performing measurements or tests in block 306 on the heart or other anatomical feature. In the illustrative embodiment, an electroanatomical map or other map is generated corresponding with the heart or organ. The map is registered to the segmented image 304 to provide a registered image 310 that may be generated and displayed as a hologram. Real-time catheter 308 data may be collected from within or about the heart using a localization technique (shape sensing, etc.). Data traces of catheter positions or other related data (treatment locations, etc.) may be rendered in a holographic image 312 which includes both the anatomical data (e.g., segmented hologram) and the device data (e.g., catheter data).

Another exemplary embodiment according to the present disclosure includes the acquisition of incomplete data (e.g., projections rather than full 3D images). This may include, for example, data in Fourier (frequency) space where intermittent or incomplete images are acquired. For example, undersampled image data in the frequency domain are collected. According to this exemplary embodiment, it is possible to construct (generate, produce, display, etc.) a 3D holographic image display with relatively less or a reduced amount of input data, and thus a relatively less or reduced amount of associated computational processing power and/or time. Depending on the incompleteness of the acquired data and what particular information may not be available, it is possible that the resultant 3D holographic image may be constructed/displayed with (some) limitations. However, such exemplary embodiments can help achieve real-time or near-real-time dynamic displays with significantly less radiation exposure (e.g., in the case of live X-ray imaging) as well as computational overhead, which benefits can be considered (e.g., balanced, weighed against) in view of the potential limitations associated with this exemplary embodiment.

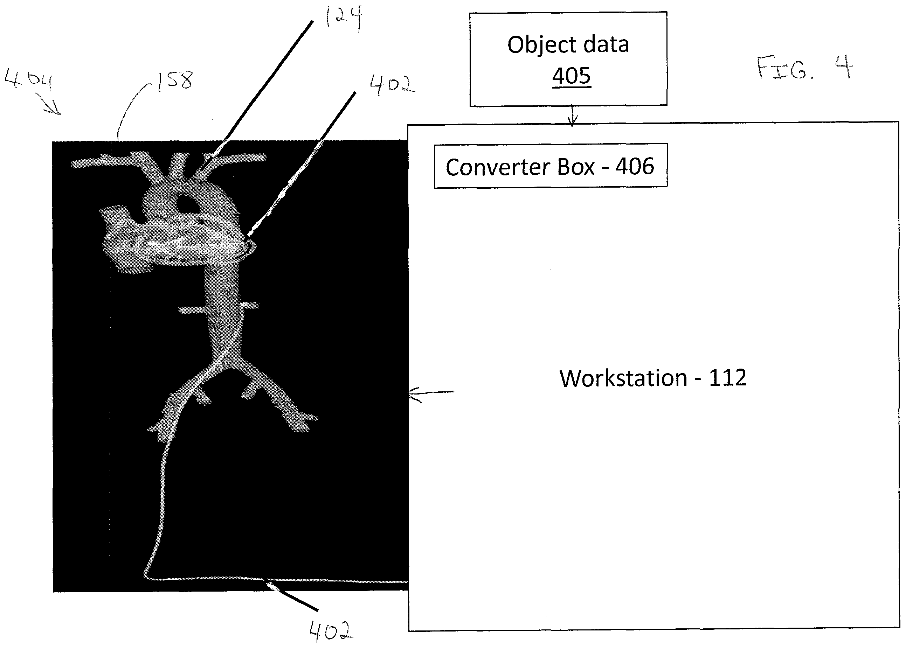

Referring to FIG. 4, another exemplary embodiment includes inputting virtual instruments or objects into a holographic display. In one embodiment, objects 402 may be digitized or otherwise rendered into a virtual environment 404 and displayed. The objects 402 may be drawn or loaded into the workstation 112 as object data 405 and may be coded into the display 158 and concurrently renders with the hologram 124. A static image of the object 402 may appear in the hologram 124 and may be separately manipulated with the hologram 124 (and or on the display 158). The static image may be employed for size comparisons or measurements between the object 402 and the hologram 124.

In one embodiment, a converter box 406 may be included to employ a standardization protocol to provide for a "video-ready" interface to the 3D holographic display 158. For example, with respect to shape sensing technology, the converter box 406 can format the x, y, z coordinates from each localized instrument or object 402 (catheter, implant, etc.) into a space readable by the holographic display 158 (e.g., rasterized/scan converted voxel space, vector space, etc.). This can be performed using the workstation 112 in FIG. 1. The 3D format should at least support voxels (for volumes), and graphical elements / primitives e.g., meshes (a virtual catheter can be displayed as a tube) and lines in 3D (to encode measurements and text rendering). The 3D format can be varied in accordance with the present disclosure based on, e.g., particular laboratory or clinical use or applications, integration with other related technologies, available resources, environmental conditions, etc. Using this video capability, the object 402 (e.g., a computer aided design rendering, model, scan, etc. for an instrument, medical device, implant, etc.) may be independently manipulated relative to the hologram 124 on the display 158 or in the air. In this way, the object 402 can be placed in or around the hologram 124 to determine whether the object will fit within a portion of the hologram 124, etc. For example, an implant may be placed through a blood vessel to test the fit visually. It is also contemplated that other feedback may be employed. For example, by understanding the space that the object 402 occupies and its orientation, a comparison module may be capable of determined interference between the hologram 124 and the objects 402 to enable, say, haptic feedback to indicate that a clearance for the implant is not possible. Other applications are also contemplated.

In another exemplary embodiment, the system 100 of FIG. 1 and/or FIG. 4 may be employed as an education and/or training tool. For example, a practitioner (e.g., surgeon, physician, fellow, doctor, etc.) could practice a procedure (surgery, case, etc.) virtually prior to actually performing the procedure by understanding the 3D anatomy and/or incorporating the use of actual or virtual tools or instruments (monitored objects 128 and/or objects 402, respectively). A fellow/practitioner could practice (perform virtually) a surgical case/procedure by, e.g., sizing an implant to plan whether it would fit a particular patient's anatomy, etc.

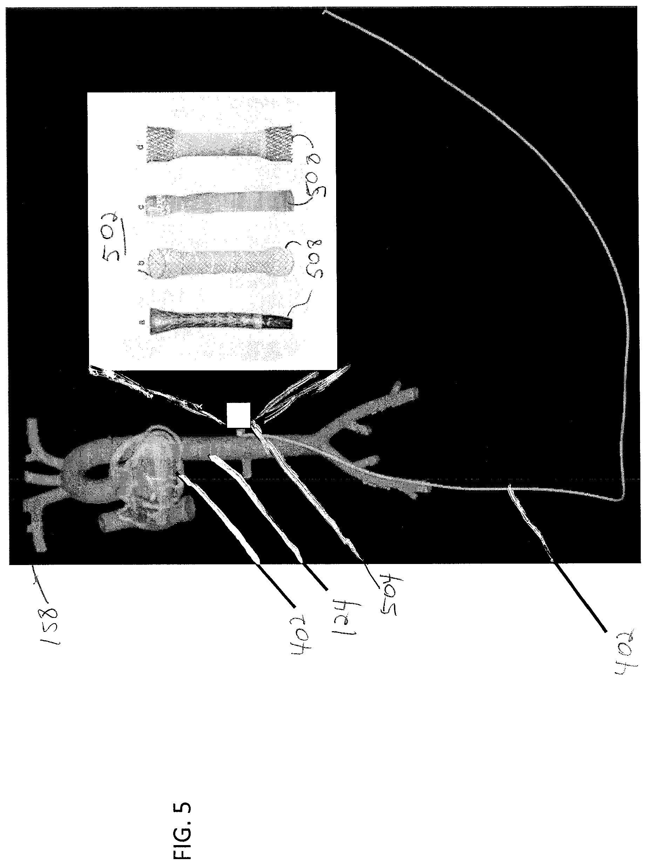

Referring to FIG. 5 with continued reference to FIG. 1, a tracked input device (monitored object 128), e.g., an instrument tracked with shape sensing, electromagnetic tracking, acoustic tracking, or machine vision based optical tracking (time-of-flight cameras or structured light cameras), may be employed in conjunction with the display 158 to access a virtual help mode trigger point 504 (or other functions) in the hologram 124 and generated by the image generation module 148. The virtual help trigger point 504 may include pixel regions within the display or hologram. For example, when manipulating virtual instruments or objects 402, the region or trigger point 504 may be selected (e.g., virtually selected and displayed by using the tip of the tracked virtual tool (402) (or using the monitored object 128) which is automatically registered with the hologram 124 in the image.

In one embodiment, the trigger points 504 are selected in the hologram 124 and a menu 502 or other interactive space may open to permit further selections. For example, a fellow/practitioner could first select a program called "HIP" by activating a trigger point 504 to display a 3D CT image of a patient's (subject's) hip, and then select different "HIP IMPLANTS" from different manufacturers to see and "feel" which implant would fit best for the particular patient. It is also possible to use (e.g., physically hold and manipulate) the actual implant in the air and position it within the 3D holographic display to see, feel and assess fit (e.g., if and how well such implant may fit the particular patient).

FIG. 5 shows the virtual menu 502 that may be provided in the holographic display 158 or other display 118 to permit the selection of a stent 508. The virtual menu 502 can be called using the display 158, the hologram 124 or by employing interface 130. Once the stent 508 is selected, a virtual model is rendered (see FIG. 4) in the display 158 or hologram 124 to permit manipulation, measurement, etc.

The virtual menu 502 provides for clinical decision support tying together localization and an exemplary holographic user interface in accordance with an exemplary embodiment. During intra-procedural use, the shape tracked instrument (128), e.g., a catheter, can be navigated to the anatomy of interest (504) and the virtual menu 502 can pop up automatically for each region, or the trigger point 504 may be activated by placing the object tip into the region of the trigger point 504 or otherwise activating the trigger point 504 (e.g., touching it, etc.). An implant or other device may be selected, which is then introduced to allow for device sizing/selection to be performed in the virtual holography space (e.g., within or in close proximity to the holographic display).

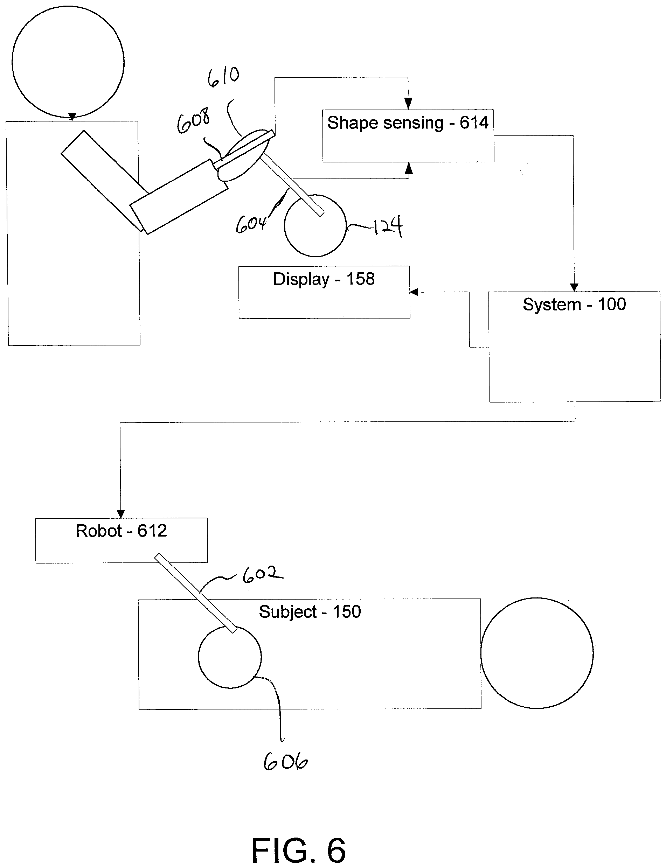

Referring to FIG. 6, according to another exemplary embodiment, a 3D holographic display 158 or hologram 124 may be employed during surgery to interact with a device 602 inside the patient. Robotics via a master/slave configuration can be used, where a shape sensed analog 604 of the device 602 moving within the display 158 is employed to actuate the motion of the actual device 602 within a target region 606. A practitioner's (surgeon's, physician's, etc.) hands 610 or voice can be tracked by sensor-based and/or voice-based techniques, such as by, e.g., tracking a physician's hands using a shape-sensing device 608 and shape sensing system 614 in the 3D holographic display. Accordingly, a practitioner's movements (including, e.g., (re)positioning, orientation, etc. of their hands) performed in the holographic display 158 can be transmitted to the device 602, such as a robot 612 (e.g., robotically controlled instruments) inside the patient to replicate such movements within the patient's body, and thereby perform the actual surgery, procedure or task inside the patient's body. Thus, a surgeon can see, touch and feel a 3D holographic display of an organ, perform a procedure thereon (i.e., within the 3D holographic display), causing such procedure to be performed inside of a patient on the actual organ via, or simply to move instruments, e.g., robotically controlled instruments.

The movement of the physician creates sensing signals using sensor 608 (and/or sensors in device 604), which are adapted to control signals by the system 100 for controlling the robot or other device 602. The signals may be stored in memory (116) for delayed execution if needed or desired. The actual procedure may be performed in real-time (or near real-time), e.g., a robot 612 performs a specific movement within a patient's body concurrently with a surgeon's performance within the 3D holographic display 158. The actual procedure may be performed by, e.g., a robot (only) after a surgeon completes a certain task/movement (or series of tasks or movements), and/or the surgeon confirms that the robot should proceed (e.g., after certain predefined criteria and/or procedural milestone(s) are reached). Such a delay (e.g., between the virtual performance of a task or movement within the 3D holographic display to the actual performance within a patient's body) can help to prevent any movements/tasks being performed within the patient incorrectly and ensure that each such movement/task is performed accurately and precisely by providing the surgeon an opportunity to confirm a movement/task after it has been performed virtually within the 3D holographic display 158 before it is actually performed within a patient's body 150 by the robot 612.

Further, a practitioner could opt to redo a specific movement/task that is virtually performed within the 3D holographic display 158 if the practitioner is not satisfied with such movement or task (for any reason). Thus, for example, if a surgeon were to inadvertently move too far in any particular direction when virtually performing a movement or task in the 3D holographic display, the surgeon could opt to redo such virtual movement or task (as many times as desired or may be necessary) until it is performed correctly. After which the actual movement or task can be performed by a robot inside of the patient with or without dynamic adaptation of the task to adjust for changes in target or therapy instrument on-the-fly (e.g., dynamically, on a continuous basis, in real-time, etc.).

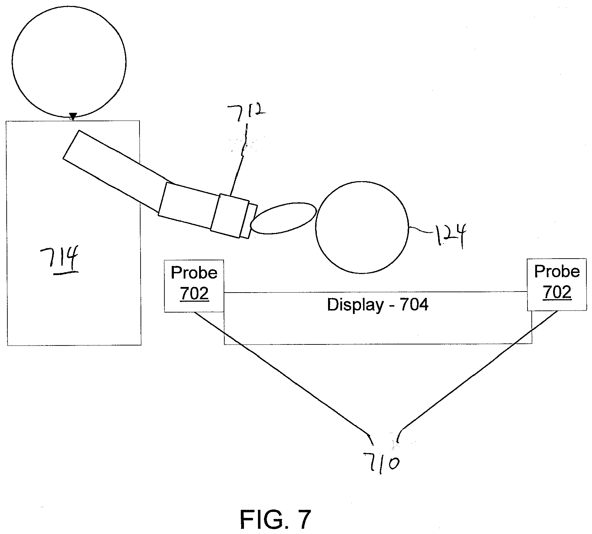

Referring to FIG. 7, another exemplary embodiment includes haptic feedback, which can be incorporated by using, e.g., ultrasound to generate vibrations in the air. A haptic device 710 may take many forms. In one embodiment, a set of ultrasonic probes 702 can be used to send customized waves towards a 3D holographic display 704 to give the user (e.g., a practitioner) a sense of feeling of structures being displayed and represented by the 3D holographic display 704. For example, such waves can be tailored or customized to represent hard or stiff materials of bony structures, while other tissues, such as of the liver and/or vessels, can be represented with a relatively softer "feel" by waves which are tailored or configured accordingly. Such encoding can be realized by, e.g., modulation of the frequency, amplitude, phase, or other spatiotemporal modulation of the excitation imparted by a haptic device 710 to the observer.

In another embodiment, the haptic feedback device 710 may be employed to, e.g., represent physical resistance of a particular structure or tissue in response to a movement or task performed by a practitioner. Thus, for example, when a surgeon 714 virtually performs a task within the 3D holographic display 704, using haptic feedback, it is possible for such task to be felt by the surgeon as if the surgeon were actually performing the task within the patient's body. This can be realized using a haptic device 712, such as, e.g., a glove(s), bracelet, or other garments or accessories having actuators or other vibratory elements.

According to one exemplary embodiment, the exemplary 3D holographic display 158 can be seen from (virtually) any angle, e.g., so that all users can interact with the same understanding and information. This is the case for an in-air hologram; however, other display techniques may be employed to provide multiple individuals with a same view.

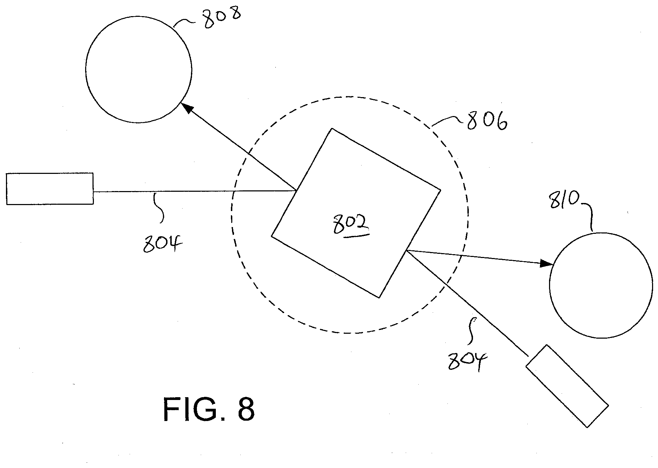

Referring to FIG. 8, display information may be provided to different users positioned in a room or area, by displaying the same or different information on a geometrical structure 802 (holographically), such as a multi-faceted holographic display where each face of the display (e.g., a cube or polyhedron) displays the information. This can be achieved by projecting multiple 2D video streams 804 on the geometrical structure 802 (e.g., side by side, or partially overlapping) rendered within a holographic output 806. For example, a holographic "cube" display in 3D can show/display on one cube face information (e.g., a 2D live x-ray image) in one particular direction (e.g., the direction of a first practitioner 808), while another cube face of the same "cube" display can show/display another type of information (e.g., an ultrasound image) to a second practitioner 810 positioned elsewhere in the room (e.g., diametrically opposite the display from the first practitioner).

One having ordinary skill in the art will appreciate in view of the teachings provided herein that such exemplary display can be configured at will depending on, e.g., the number of users in the room. It is also possible that the position (in the room) of each user/practitioner can be tracked (in the room) and that each individual's display information follows each user's viewing perspective as the user moves (e.g., during a procedure). For example, one particular user (doctor, nurse, etc.) can be provided with the specific information that the user needs regardless of where in the room such particular user moves during a procedure. Further, it is also possible that each user is provided with a unique display, which can be a 2D cube face, such as described above, or a 3D holographic display customized or tailored for such user, and that such a unique display can "follow" the user as the user moves around a room.

Multiple combinations of displays in accordance with this and other exemplary embodiments described herein are possible, providing, e.g., for individual users to have their own unique display and/or be presented with the same information of other users, regardless of the movement and location of a user within a room or elsewhere (e.g., outside of the room, off-site, etc.). Additionally, a user may initially select and change at any time during a procedure what information is displayed to them by, e.g., selecting from predefined templates, selecting specific informational fields, selecting the display of another particular user, etc.

Note that text is an inherently 2D mode of communication. The system may display shapes/symbols identifiable from multiple viewpoints, or represent the text oriented towards the viewer. In case of multiple viewers, the oriented text may be shown in multiple directions simultaneously or to each independently in different frames.

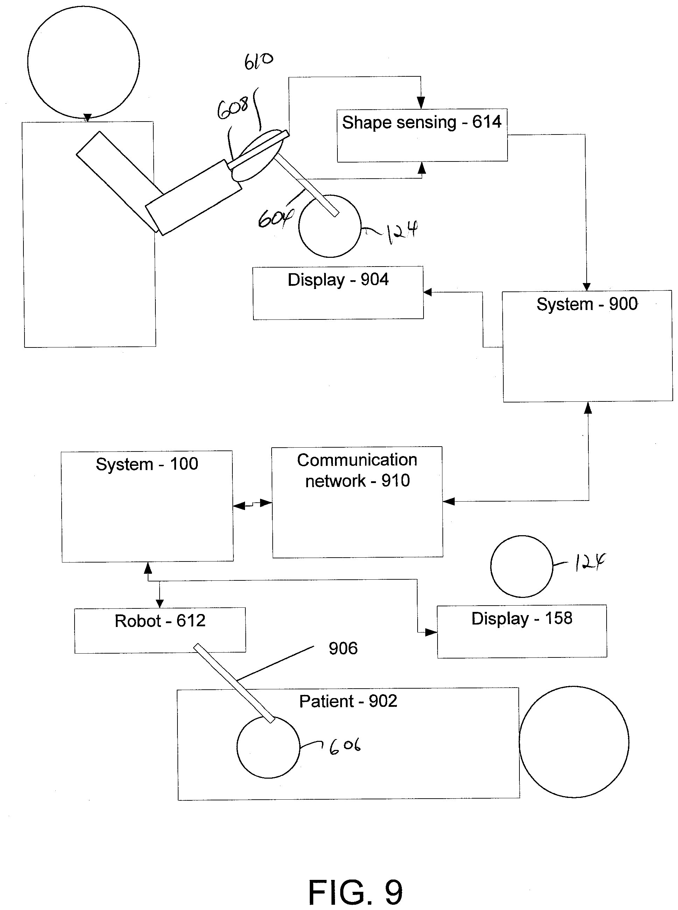

Referring to FIG. 9, in another exemplary embodiment, a remote system 900 may include at least some of the capabilities of system 100 (FIG. 1) but is remotely disposed relative to a patient 902 and data collection instruments. A user (practitioner, surgeon, fellow, etc.) may conduct a procedure remotely (e.g., with the user being physically located off-site from the location where the subject/patient 902 is located) or assist or provide guidance remotely to the procedure. For example, a user can perform a procedure/task on an exemplary holographic display 904 located at their location. In one embodiment, the display 904 is connected (e.g., via the Internet or other network 910 (wired or wireless)) to the system 100 co-located with the patient 902. System 100 can be in continuous communication with the remote system 900 (e.g., where the user is located) so that the holographic display 904 is continually updated in (near) real-time. Additionally, the system 100 may include robotically controlled instruments 906, e.g., inside of a patient) which are controlled via commands provided (e.g., via the Internet) by the remote system 900, as described above. These commands are generated based on the user's interaction with the holographic display 904. Holographic displays 158 and 904 may include the same subject matter at one or more locations so that the same information is conveyed at each location. For example, this embodiment may include, e.g., providing guidance or assistance to another doctor around the globe, for peer-to-peer review, expert assistance or a virtual class room where many students could attend a live case from different locations throughout the world.

Some or all of the exemplary embodiments and features described herein can also be used (at least in part) in conjunction or combination with any other embodiments described herein.

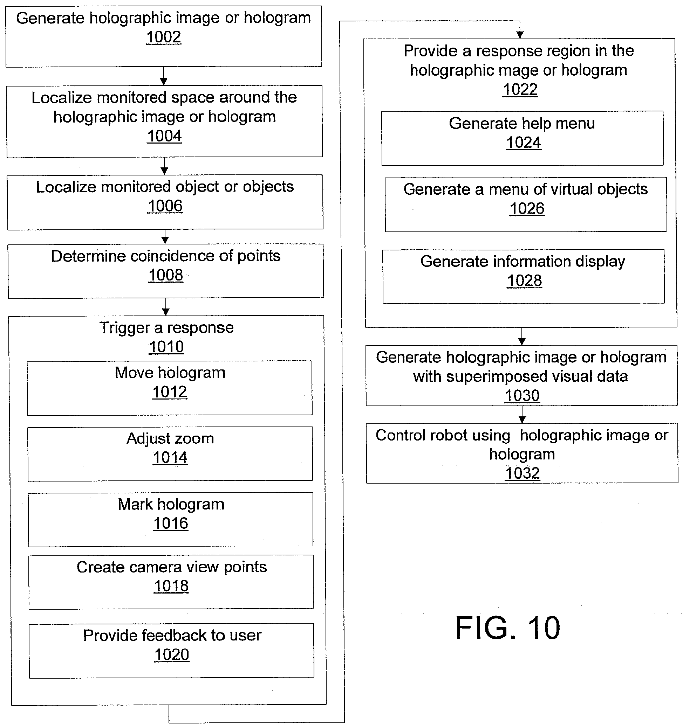

Referring to FIG. 10, a method for interacting with a holographic display is shown in accordance with illustrative embodiments. In block 1002, a holographically rendered anatomical image is generated and displayed. The image may include one or more organs or anatomical regions. The holographically rendered anatomical image may be generated in-air.

In block 1004, a monitored space is localized on or around the holographically rendered anatomical image to define a region for interaction. The localization system may include one or more of a fiber optic shape sensing system, an electromagnetic tracking system, a light sensor array and/or other sensing modality. The position and orientation of the monitored space and the one or more monitored objects is preferably determined in a same coordinate system. The one or more monitored objects may include a medical instrument, an anatomical feature of a user, a virtual object, etc.

In block 1006, a position and orientation of one or more monitored objects is monitored by the localization system. In block 1008, coincidence of spatial points is determined between the monitored space the one or more monitored objects. In block 1010, if coincidence is determined, a response is triggered in the holographically rendered anatomical image. In block 1012, the response may include moving the holographically rendered anatomical image (e.g. 6DOF) or changing its appearance. In block 1014, the response may include adjusting a zoom (magnification) or other optical characteristics of the holographically rendered anatomical image. In block 1016, the holographically rendered anatomical image may be marked, tagged, targeted, etc. In block 1018, camera viewpoints can be assigned (for other viewers or displays). In block 1020, feedback may be generated to a user. The feedback may include haptic feedback (vibrating device or air), optical feedback (visual or color differences), acoustic feedback (verbal, alarms), etc.

In block 1022, a response region may be provided and monitored by the localization system such that upon activating the response region a display event occurs. The display event may include generating a help menu in block 1024; generating a menu of virtual objects to be included in the holographically rendered anatomical image upon selection in block 1026; and generating information to be displayed in block 1028.

In block 1030, the holographically rendered anatomical image may be generated with superimposed medical data mapped to positions on the holographically rendered anatomical image. In block 1032, the response that is triggered may include generating control signals for operating robotically controlled instruments. The control signals may enable remote operations to be performed.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for holographic user interfaces for medical procedures (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. An interactive holographic display system, comprising:
a holographic generation module (115) configured to display a holographically rendered anatomical image (124);
a localization system (120) configured to define a monitored space (126) on or around the holographically rendered anatomical image; and
one or more monitored objects (128) having their position and orientation monitored by the localization system such that coincidence of spatial points between the monitored space and the one or more monitored objects triggers a response in the holographically rendered anatomical image, wherein the response includes a haptic feedback to a user, the haptic feedback being incorporated by using ultrasound to generate vibrations in the air,
**characterized in that** the localization system (120) includes a shape sensing system for performing shape sensing of the one or more monitored objects (128),
wherein the shape sensing system is a fiber optic based shape sensing system configured to include one or more optical fibers coupled to the one or more monitored objects (128),
wherein a plurality of sensor elements are distributed over the length of the one or more optical fibers, wherein three or more fiber cores with the plurality of sensor elements are incorporated along the length of the one or more optical fibers embedded in the one or more monitored objects (128) for determining a three-dimensional form of the one or more monitored objects (128).

2. The system as recited in claim 1, wherein a plurality of Bragg gratings or an inherent backscatter, such as a Rayleigh scatter, are used to form the plurality of sensor elements of the fiber optic based shape sensing system.

3. The system as recited in claim 2, wherein random fluctuations of an index of refraction in a fiber core are modeled as a Bragg grating with a random variation of amplitude and phase along the grating length.

4. The system as recited in claim 1, wherein the response uses a haptic device (710) comprising a set of ultrasonic probes (702) to send customized waves towards a 3D holographic display (704), and wherein the one or more monitored objects (128) include a medical instrument, an anatomical feature of a user and a virtual object.

5. The system as recited in claim 4, wherein the waves are configured to represent hard or stiff materials of bony structures and/or tissues of the liver and/or vessels.

6. The system as recited in claim 4, wherein modulation of the frequency, amplitude, phase, or other spatiotemporal modulation of the excitation imparted by the haptic device (710) to an observer is realized.

7. The system as recited in claim 1, wherein the response in the holographically rendered anatomical image (124) includes one or more of: translation or rotation of the holographically rendered anatomical image, magnification adjustment of the holographically rendered anatomical image, marking of the holographically rendered anatomical image and feedback generation.

8. The system as recited in claim 1, wherein the holographically rendered anatomical image (124) includes a response region (504) monitored by the localization system such that upon activating the response region a display event (502) occurs, and/or wherein the holographically rendered anatomical image (124) displays superimposed medical data mapped to positions thereon.

9. The system as recited in claim 8, wherein the display event (502) includes a generated help menu, a generated menu of virtual objects to be included in the holographically rendered anatomical image upon selection and a generated information display.

10. The system as recited in claim 1, wherein the response in the holographically rendered anatomical image (124) generates control signals for operating robotically controlled instruments (602) and/or includes seed points (162) placed to direct virtual camera angles for an additional display.

11. The system as recited in claim 1, wherein the interactive holographic display system is remotely disposed from a patient location and connected to the patient location over a communication network (910), and/or wherein the interactive holographic display system is remotely disposed from a patient location and connected to the patient location over a communication network (910) such that the holographically rendered anatomical image is employed to remotely control instruments (906) at the patient's location.

12. The system as recited in claim 1, comprising:
a processor (114);
memory (116) coupled to the processor;
wherein the holographic generation module (115) is included in the memory and is configured to display the holographically rendered anatomical image (124) as an in-air hologram or on a holographic display;
wherein the response in the holographically rendered anatomical image includes one or more of: translation or rotation of the holographically rendered anatomical image, magnification adjustment of the holographically rendered anatomical image, marking of the holographically rendered anatomical image and feedback generation.

13. A method for interacting with a holographic display, comprising:
displaying (1002) a holographically rendered anatomical image;
localizing (1004) a monitored space on or around the holographically rendered anatomical image to define a region for interaction;
monitoring (1006) a position and orientation of one or more monitored objects by the localization system;
determining (1008) coincidence of spatial points between the monitored space the one or more monitored objects; and
if coincidence is determined, triggering (1010) a response in the holographically rendered anatomical image, wherein the response includes a haptic feedback to a user, the haptic feedback being incorporated by using ultrasound to generate vibrations in the air,
**characterized in that** the localizing comprises using a shape sensing system to perform shape sensing of the one or more monitored objects (128),
wherein the shape sensing system is a fiber optic based shape sensing system configured to include one or more optical fibers coupled to the one or more monitored objects (128),
wherein a plurality of sensor elements are distributed over the length of the one or more optical fibers, wherein three or more fiber cores with the plurality of sensor elements are incorporated along the length of the one or more optical fibers embedded in the one or more monitored objects (128) for determining a three-dimensional form of the one or more monitored objects (128).

## Patentansprüche

1. Interaktives holografisches Anzeigesystem, umfassend:
ein holografisches Erzeugungsmodul (115), das konfiguriert ist, um ein holografisch gerendertes anatomisches Bild (124) anzuzeigen;
ein Lokalisierungssystem (120), das konfiguriert ist, um einen überwachten Raum (126) auf oder um das holografisch gerenderte anatomische Bild zu definieren; und
ein oder mehrere überwachte Objekte (128), deren Position und Ausrichtung durch das Lokalisierungssystem überwacht wird, sodass Koinzidenz von räumlichen Punkten zwischen dem überwachten Raum und dem einen oder mehreren überwachten Objekten eine Reaktion in dem holografisch gerenderten anatomischen Bild auslöst, wobei die Reaktion eine haptische Rückmeldung an einen Benutzer umfasst, wobei die haptische Rückmeldung durch Nutzen von Ultraschall zum Erzeugen von Schwingungen in der Luft verkörpert ist,
**dadurch gekennzeichnet, dass** das Lokalisierungssystem (120) ein Formerfassungssystem zum Durchführen einer Formerfassung von dem einen oder mehreren überwachten Objekten (128) umfasst,
wobei das Formerfassungssystem ein auf Faseroptik basierendes Formerfassungssystem ist, das konfiguriert ist, um eine oder mehrere optische Fasern zu umfassen, die mit dem einen oder mehreren überwachten Objekten (128) gekoppelt sind,
wobei eine Vielzahl von Sensorelementen über die Länge der einen oder mehreren optischen Fasern verteilt sind, wobei drei oder mehr Faserkerne mit der Vielzahl von Sensorelementen entlang der Länge der einen oder mehreren optischen Fasern, die in das eine oder mehrere überwachte Objekte (128) eingebettet sind, aufgenommen sind, um eine dreidimensionale Form von dem einen oder mehreren überwachten Objekten (128) zu bestimmen.

2. System nach Anspruch 1, wobei eine Vielzahl von Bragg-Gittern oder eine inhärente Rückstreuung, zum Beispiel ein Rayleigh-Streuung, verwendet werden, um die Vielzahl von Sensorelementen des auf Faseroptik basierenden Formerfassungssystems zu bilden.

3. System nach Anspruch 2, wobei Zufallsschwankungen eines Brechungsindex in einem Faserkern als ein Bragg-Gitter mit einer Zufallsveränderung von Amplitude und Phase entlang der Gitterlänge modelliert werden.

4. System nach Anspruch 1, wobei die Reaktion eine haptische Vorrichtung (710) umfassend einen Satz von Ultraschallsonden (702) nutzt, um angepasste Wellen zu einer holografischen 3D-Anzeige (704) zu senden, und wobei das eine oder mehrere überwachte Objekte (128) ein medizinisches Instrument, ein anatomisches Merkmal eines Benutzers oder ein virtuelles Objekt umfassen.

5. System nach Anspruch 4, wobei die Wellen konfiguriert sind, um harte oder steife Materialien von knochiger Struktur und/oder Gewebe der Leber und/oder Gefäße darzustellen.

6. System nach Anspruch 4, wobei eine Modulation der Frequenz, Amplitude, Phase oder eine andere räumlich-zeitliche Modulation der Anregung, die dem Benutzer durch die haptische Vorrichtung (710) mitgeteilt wird, realisiert wird.

7. System nach Anspruch 1, wobei die Reaktion in dem holografisch gerenderten anatomischen Bild (124) eines oder mehreres von Folgendem umfasst:
Translation oder Rotation des holografisch gerenderten anatomischen Bilds, Vergrößerungsanpassung des holografisch gerenderten anatomischen Bilds, Kennzeichnung des holografisch gerenderten anatomischen Bilds und Rückmeldungserzeugung.

8. System nach Anspruch 1, wobei das holografisch gerenderte anatomische Bild (124) eine Reaktionsregion (504) umfasst, die durch das Lokalisierungssystem überwacht wird, sodass bei Aktivierung der Reaktionsregion ein Anzeigeereignis (502) auftritt, und/oder wobei das holografisch gerenderte anatomische Bild (124) überlagerte Daten anzeigt, die auf Positionen darauf abgebildet sind.

9. System nach Anspruch 8, wobei das Anzeigeereignis (502) ein erzeugtes Hilfemenü, ein erzeugtes Menü virtueller Objekte, das bei Auswahl in das holografisch gerenderte anatomische Bild einzuschließen ist, und eine erzeugte Informationsanzeige umfasst.

10. System nach Anspruch 1, wobei die Reaktion in dem holografisch gerenderten anatomischen Bild (124) Steuersignale zum Betreiben von robotergesteuerten Instrumenten (602) erzeugt und/oder Saatpunkte (162) umfasst, die platziert werden, um virtuelle Kamerawinkel für eine zusätzliche Anzeige zu lenken.

11. System nach Anspruch 1, wobei das interaktive holografische Anzeigesystem entfernt von einem Patientenort angeordnet ist und über ein Kommunikationsnetzwerk (910) mit dem Patientenort verbunden ist, und/oder wobei das interaktive holografische Anzeigesystem entfernt von einem Patientenort angeordnet ist und über ein Kommunikationsnetzwerk (910) derartig mit dem Patientenort verbunden ist, dass das holografisch gerenderte anatomische Bild verwendet wird, um Instrumente (906) am Patientenort aus der Ferne zu steuern.

12. System nach Anspruch 1, umfassend:
einen Prozessor (114);
mit dem Prozessor gekoppelten Speicher (116);
wobei das holografische Erzeugungsmodul (115) in dem Speicher enthalten ist und konfiguriert ist, um das holografisch gerenderte anatomische Bild (124) als ein Hologramm in der Luft oder auf einer holografischen Anzeige anzuzeigen;
wobei die Reaktion des holografisch gerenderten anatomischen Bilds eines oder mehreres von Folgendem umfasst: Translation oder Rotation des holografisch gerenderten anatomischen Bilds, Vergrößerungsanpassung des holografisch gerenderten anatomischen Bilds, Kennzeichnung des holografisch gerenderten anatomischen Bilds und Rückmeldungserzeugung.

13. Verfahren zum Interagieren mit einer holografischen Anzeige, umfassend:
Anzeigen (1002) eines holografisch gerenderten anatomischen Bilds;
Lokalisieren (1004) eines überwachten Raums auf oder um das holografisch gerenderte anatomische Bild, um eine Region zur Interaktion zu definieren; und
Überwachen (1006) einer Position und Ausrichtung von einem oder mehreren überwachten Objekten durch das Lokalisierungssystem;
Ermitteln (1008) von Koinzidenz von räumlichen Punkten zwischen dem überwachten Raum des einen oder mehrerer überwachter Objekte; und
wenn Koinzidenz festgestellt wird, Auslösen (1010) einer Reaktion in dem holografisch gerenderten anatomischen Bild, wobei die Reaktion eine haptische Rückmeldung an einen Benutzer umfasst, wobei die haptische Rückmeldung durch Nutzen von Ultraschall zum Erzeugen von Schwingungen in der Luft verkörpert ist,
**dadurch gekennzeichnet, dass** die Lokalisierung das Verwenden eines Formerfassungssystems zum Durchführen einer Formerfassung von dem einen oder mehreren überwachten Objekten (128) umfasst,
wobei das Formerfassungssystem ein auf Faseroptik basierendes Formerfassungssystem ist, das konfiguriert ist, um eine oder mehrere optische Fasern zu umfassen, die mit dem einen oder mehreren überwachten Objekten (128) gekoppelt sind,
wobei eine Vielzahl von Sensorelementen über die Länge der einen oder mehreren optischen Fasern verteilt sind, wobei drei oder mehr Faserkerne mit der Vielzahl von Sensorelementen entlang der Länge der einen oder mehreren optischen Fasern, die in das eine oder mehrere überwachte Objekte (128) eingebettet sind, aufgenommen sind, um eine dreidimensionale Form von dem einen oder mehreren überwachten Objekten (128) zu bestimmen.

## Revendications

1. Système d'affichage holographique interactif comprenant :
un module de génération holographique (115) configuré pour afficher une image anatomique à rendu holographique (124) ;
un système de localisation (120) configuré pour définir un espace surveillé (126) sur l'image anatomique à rendu holographique ou autour de celle-ci ; et
un ou plusieurs objets surveillés (128) ayant leur position et leur orientation surveillées par le système de localisation de sorte que la coïncidence de points spatiaux entre l'espace surveillé et les un ou plusieurs objets surveillés déclenche une réponse anatomique à rendu holographique, dans lequel la réponse comprend une rétroaction haptique à un utilisateur, la rétroaction haptique étant incorporée par l'utilisation d'ultrasons pour générer des vibrations dans l'air,
**caractérisé en ce que** le système de localisation (120) comprend un système de détection de forme pour effectuer une détection de forme des un ou plusieurs objets surveillés (128),
dans lequel le système de détection de forme est un système de détection de forme à base de fibre optique configuré pour comprendre une ou plusieurs fibres optiques couplées aux un ou plusieurs objets surveillés (128),
dans lequel une pluralité d'éléments de capteur est distribuée sur la longueur des une ou plusieurs fibres optiques, dans lequel trois ou plus de noyaux de fibres avec la pluralité d'éléments de capteurs sont incorporés sur la longueur des une ou plusieurs fibres optiques noyées aux un ou plusieurs objets surveillés (128) pour déterminer une forme tridimensionnelle des un ou plusieurs objets surveillés (128).

2. Système selon la revendication 1, dans lequel une pluralité de réseaux de Bragg ou un rétrodiffuseur inhérent, tel qu'un diffuseur de Rayleigh, est utilisée pour former la pluralité des éléments de capteurs du système de détection de forme à base de fibre optique.

3. Système selon la revendication 2, dans lequel des fluctuations aléatoires d'un indice de réfraction dans un noyau de fibre sont modélisées comme réseau de Bragg avec une variation aléatoire de l'amplitude et de la phase sur la longueur du réseau.

4. Système selon la revendication 1, dans lequel la réponse utilise un dispositif haptique (710) comprenant un ensemble de sondes ultrasoniques (702) pour envoyer des ondes personnalisées vers un affichage holographique (704) et
dans lequel les un ou plusieurs objets surveillés (128) comprennent un instrument optique, une caractéristique anatomique d'un utilisateur et un objet virtuel.

5. Système selon la revendication 4, dans lequel les ondes sont configurées pour représenter des matériaux durs ou rigides de structures osseuses et/ou des tissus du foie et/ou des vaisseaux.

6. Système selon la revendication 4, dans lequel une modulation de la fréquence, de l'amplitude, de la phase ou une autre modulation spatiotemporelle de l'excitation communiquée par le dispositif haptique (710) à un observateur est réalisée.

7. Système selon la revendication 1, dans lequel la réponse dans l'image anatomique à rendu holographique (124) comprend une ou plusieurs d'une translation ou d'une rotation de l'image anatomique à rendu holographique, d'un ajustement du grossissement de l'image anatomique à rendu holographique, d'un marquage de l'image anatomique à rendu holographique et d'une génération de rétroaction.

8. Système selon la revendication 1, dans lequel l'image anatomique à rendu holographique (124) comprend une région de réponse (504) surveillée par le système de localisation en sorte que, lors de l'activation de la région de réponse, un événement d'affichage (502) se produise, et/ou dans lequel l'image anatomique à rendu holographique (124) affiche des données médicales superposées mappées à des positions sur celle-ci.

9. Système selon la revendication 8, dans lequel l'événement d'affichage (502) comprend un menu d'aide généré, un menu généré d'objets virtuels à inclure dans l'image anatomique à rendu holographique lors de la sélection et un affichage d'informations générées.

10. Système selon la revendication 1, dans lequel la réponse dans l'image anatomique à rendu holographique (124) génère des signaux de commande pour activer des instruments commandés par voie robotique (602) et/ou comprend des points d'ensemencement (162) placés pour diriger des angles de caméra virtuels pour un affichage supplémentaire.

11. Système selon la revendication 1, dans lequel le système d'affichage holographique interactif est disposé à distance d'un emplacement de patient et est raccordé à l'emplacement de patient via un réseau de communication (910) et/ou dans lequel le système d'affichage holographique est disposé à distance d'un emplacement de patient et raccordé à l'emplacement de patient via un réseau de communication (910) tel que l'image anatomique à rendu holographique soit utilisée pour commander à distance des instruments (906) à l'emplacement du patient.

12. Système selon la revendication 1, comprenant :
un processeur (114) ;
une mémoire (116) couplée au processeur ;
dans lequel le module de génération holographique (115) est compris dans la mémoire et est configuré pour afficher l'image anatomique à rendu holographique (124) comme un hologramme dans l'air ou sur un affichage holographique ;
dans lequel la réponse dans l'image anatomique à rendu holographique comprend une ou plusieurs d'une translation ou d'une rotation de l'image anatomique à rendu holographique, d'un ajustement du grossissement de l'image anatomique à rendu holographique, d'un marquage de l'image anatomique à rendu holographique et d'une génération de rétroaction.

13. Procédé pour interagir avec un affichage holographique, comprenant :
l'affichage (1002) d'une image anatomique à rendu holographique ;
la localisation (1004) d'un espace surveillé sur l'image anatomique à rendu holographique ou autour de celle-ci afin de définir une région pour l'interaction ;
la surveillance (1006) d'une position et d'une orientation des un ou plusieurs objets surveillés par le système de localisation ;
la détermination (1008) d'une coïncidence de points spatiaux entre l'espace surveillé d'un ou plusieurs objets surveillés ; et,
si une coïncidence est déterminée, le déclenchement (1010) d'une réponse dans l'image anatomique à rendu holographique, dans lequel la réponse comprend une rétroaction haptique à un utilisateur, la rétroaction haptique étant incorporée en utilisant des ultrasons pour générer des vibrations dans l'air,
**caractérisé en ce que** la localisation comprend l'utilisation d'un système de détection de forme pour effectuer une détection de forme des un ou plusieurs objets surveillés (128),
dans lequel le système de détection de forme est un système de détection de forme à base de fibre optique configuré pour comprendre une ou plusieurs fibres optiques couplées aux un ou plusieurs objets surveillés (128),
dans lequel une pluralité d'éléments de capteur est distribuée sur la longueur des une ou plusieurs fibres optiques, dans lequel trois ou plusieurs noyaux de fibres avec la pluralité d'éléments de capteur sont incorporés sur la longueur des une ou plusieurs fibres optiques noyées dans les un ou plusieurs objets surveillés (128) pour déterminer une forme tridimensionnelle des un ou plusieurs objets surveillés (128).
